**Europäisches Patentamt**

**European Patent Office**

**Office européen des brevets**

(19)

(11) Publication number : **0 640 388 A1**

(12) # EUROPEAN PATENT APPLICATION

(21) Application number : **94306228.1**

(22) Date of filing : **24.08.94**

(51) Int. Cl.$^6$ : **B01J 21/10,** C07C 213/06, B01J 37/08

(30) Priority : **25.08.93 US 112583**

(43) Date of publication of application :
**01.03.95 Bulletin 95/09**

(84) Designated Contracting States :
**BE CH DE ES FR GB IT LI SE**

(71) Applicant : **UNION CARBIDE CHEMICALS & PLASTICS TECHNOLOGY CORPORATION
39 Old Ridgebury Road
Danbury, Connecticut 06817-0001 (US)**

(72) Inventor : **King, Stephen Wayne
120 Teays Meadows
Scott Depot, West Virginia 25560 (US)**
Inventor : **Mierau, Stefan Kent
379 Central Avenue
South Charleston, West Virginia 25303 (US)**

(74) Representative : **Allard, Susan Joyce et al
BOULT, WADE & TENNANT
27 Furnival Street
London EC4A 1PQ (GB)**

(54) **Magnesium/aluminum mixed oxide catalysts and their use in the production of aminoethers.**

(57)   Calcined magnesium/aluminum mixed oxide catalysts derived from water-insoluble magnesium and aluminum carbonate containing salts, which catalysts are particularly useful in the production of aminoethers, especially bis(2-dimethylaminoethyl) ether.

EP 0 640 388 A1

## BACKGROUND OF THE INVENTION

### Field of the Invention

This invention relates to novel calcined magnesium/aluminum mixed oxide catalysts derived from certain water-insoluble magnesium and aluminum carbonate containing salts, which have been found to be particularly effective catalysts for use in the production of aminoethers, especially bis(2-dimethylaminoethyl) ether.

### Background Art

Calcined mixed metal oxide compositions, including various different calcined magnesium/aluminum oxide compositions have been promoted as catalysts for the production of aminoethers, see e.g. USP 5,214,142, as well as for numerous other base catalyzed type reactions. It is also known in the art that any two given calcined mixed metal oxide catalysts may in actuality be completely different compositions of matter even though they may have the same metals, said difference being evidenced by the obtaining of highly different comparative catalyst performance results for each catalyst. However, since such different calcined mixed metal oxide catalysts may not be readily delineated from each other by the use of simple formulas, it has become common place in the art to distinguish such catalysts from each other by defining them in terms of how the particular catalysts are derived.

For instance, one type of mixed metal oxide catalysts disclosed in USP 5,214,142 are calcined catalysts derived from hydrotalcite type compositions of magnesium-aluminum hydroxide carbonate, prepared e.g. by coprecipitation of an aqueous solution of water-soluble metal salts, e.g. nitrates, sulfates or chlorides of magnesium and aluminum in the presence of an alkali metal base (e.g. sodium hydroxide), and a water soluble salt of a desired anion (e.g. sodium carbonate) followed by heating the precipitated slurry to produce the hydrotalcite type complex which is then dried and calcined to obtain the desired catalyst. However, the calcined magnesium/aluminum mixed oxide catalysts of this invention are clearly far removed from catalysts prepared by such coprecipitation techniques. Moreover, it is inherently difficult to reproduce catalysts having the exact or nearly the same beneficial properties by such coprecipitation type procedures. For instance, in addition to having to carefully monitor and correlate four different water-soluble ingredients, common adverse precipitation and/or filtration type problems may arise particularly during large scale type productions of such catalysts. U.S.P. 5,214,142 also discloses obtaining a magnesium/aluminum mixed oxide catalyst by mixing magnesium carbonate hydroxide pentahydrate and an aluminum hydroxide hydrate with deionized water to form a paste which is then calcined at 350°C to 450°C to obtain the desired catalyst. However, said patent does not teach the use of the specific water-insoluble aluminum salt employed in the subject invention.

It has now been discovered that such adverse coprecipitation type procedures, heretofore employed in producing calcined magnesium/aluminum mixed oxide catalysts from hydrotalcite type complex intermediates, can be avoided by deriving the calcined magnesium/aluminum mixed oxide catalysts from an aqueous mixture of a water-insoluble magnesium carbonate compound and a water-insoluble aluminum hydroxide carbonate hydrate. The use of such water-insoluble metal carbonate salts of this invention allows for the production of calcined magnesium/aluminum mixed oxide catalysts via efficient and simple catalyst production procedures, and said catalysts have been found to provide very good desired aminoether product production selectivities over prolonged periods of continuous operation.

### Disclosure of the Invention

Thus it is an object of this invention to provide novel calcined magnesium/aluminum mixed oxide catalysts which may be produced by a different and/or far simpler catalyst production procedure than heretofore disclosed in the prior art, and which are especially useful for the production of aminoethers. Another object of this invention is to provide a process for preparing aminoethers which comprises decarboxylating an aminoether carboxylate compound by contacting it with a calcined magnesium/aluminum mixed oxide catalyst of this invention under conditions effective to produce the aminoether. Other objects and advantages of this invention will become readily apparent from the following written description and appended claims.

Accordingly, a generic aspect of this invention can be described as a calcined magnesium/aluminum mixed oxide catalyst derived from an aqueous mixture consisting of from about 0.1 to about 5 parts by weight of a water-insoluble magnesium carbonate selected from the group consisting of magnesium carbonate hydroxide pentahydrate, anhydrous magnesium carbonate, magnesium carbonate monohydrate, and magnesium carbonate hydroxide trihydrate, per one part by weight of a water-insoluble aluminum hydroxide carbonate hydrate, and from about 1 to about 10 parts by weight of water per one part of the total weight of said water-insoluble

2

aluminum and magnesium compounds employed.

Of course it is to be understood that, if desired, the water-insoluble magnesium carbonate employed herein could consist of a mixture of any two or more of said recited magnesium carbonate compounds, although it is generally preferred to employ only one magnesium carbonate compound at a time. Moreover as used herein in the catalyst definition of this invention, the term "oxide" embraces oxides, hydroxides, and/or mixtures thereof.

Detailed Description

As noted above the calcined magnesium/aluminum mixed oxide catalysts of this invention are derived from an aqueous mixture consisting of from about 0.1 to about 5 parts by weight, preferably from about 0.5 to about 5 parts by weight of the water-insoluble magnesium carbonate per one part by weight of the water-insoluble aluminum hydroxide carbonate hydrate, and from about 1 to about 10 parts by weight, preferably from about 1 to about 5 parts by weight of water per one part of the total weight of said water-insoluble aluminum and magnesium compounds employed. More preferably said aqueous mixtures consist of from about 1 to about 3 parts by weight of the water-insoluble magnesium carbonate per one part by weight of the water-insoluble aluminum hydroxide carbonate hydrate, along with from about 1 to about 3 parts by weight of water per one part of the total weight of said water-insoluble aluminum and magnesium compounds employed. Moreover said aqueous mixtures are preferably employed in the form of a smooth paste made by simply thoroughly mixing the two solid water-insoluble magnesium and aluminum salts with water under ambient conditions such as at about room temperature and in any suitable manner desired. Of course, it is to be understood that while said aqueous mixtures (or paste) must contain a minimum amount of about 1 part by weight of water per one part of the total weight of said water-insoluble aluminum and magnesium compounds employed, the upper limit on the amount of water present is not critical and need only be governed by obvious practical constraints and common sense. Any excess or undesirable amounts of water present may be readily filtered out of the aqueous mixture or paste prior to employing it as taught here. It is to be further understood that the aqueous mixture starting material or paste can be the result of mixing dry water-insoluble aluminum and magnesium salts with water or by combining aqueous or wet slurries of said salts along with additional added water if necessary. Preferably distilled or deionized water is employed, although such may not be absolutely necessary.

The water-insoluble aluminum hydroxide carbonate hydrate compounds employable herein may be obtained from Aldrich Chemical Co., Inc. under the designation aluminum hydroxide hydrate, powder or from Chattem Chemicals under such designations as aluminum hydroxide gel and aluminum hydroxide gel, dried. Such materials comprise an amorphous hydrated form of aluminum hydroxide in which there is a partial substitution of carbonate for hydroxide as explained e.g. on page 52 of The United States Pharmacopeia, Twenty-Second Revision, (USP XXII; NF XVII), 1990.

Illustrative water-insoluble magnesium carbonate compounds that may be employable in this invention, along with companies that supply same, include magnesium carbonate hydroxide pentahydrate (Aldrich Chemical Co., Inc.), anhydrous magnesium carbonate (Eastman Fine Chemicals), magnesium carbonate monohydrate (J. T. Baker Inc.) and magnesium carbonate hydroxide trihydrate (Pfaltz and Bauer Inc.). The preferred magnesium carbonate compound is magnesium carbonate hydroxide pentahydrate.

Accordingly a unique feature of the subject invention, and one that is considered critical to the excellent performance of the calcined magnesium/aluminum mixed oxide catalysts for selectively producing aminoethers, especially bis(2-dimethylaminoethyl) ether, over prolonged periods of time, is the presence of carbonate in both the magnesium and aluminum starting materials. The absence of such carbonate in either starting material has been found to result in catalysts that do not provide as good an aminoether product selectivity over comparable catalytic activity life spans as the catalysts of this invention.

If desired, calcined magnesium/aluminum mixed oxide catalyst powders of this invention may be readily obtained by directly calcining the above-described aqueous mixtures or pastes of said water-insoluble magnesium and aluminum salts at any suitable temperature, e.g. from about 250°C to about 800°C and preferably from about 300°C to about 500°C, for a sufficient period of time to produce the desired catalyst, in any conventional known manner. In general periods of time ranging from about 2 to about 24 hours should be sufficient to complete most calcinations. Moreover the calcination (or heat) treatment may be carried out in any suitable furnace or oven e.g. a muffle furnace, or in air or an inert gas stream or even under vacuum. Of course it is to be understood that too low or too high a calcination temperature should be avoided for obvious conventional reasons and that optimum calcination temperatures may be readily determined by routine experimentation. In general it may be preferred to employ calcination temperatures which maximize the catalyst surface area and pore volume of the amorphous like calcined magnesium/aluminum mixed oxide product. The calcined magnesium/aluminum mixed oxide catalyst powders so obtained may be employed directly in their powdered form, e.g. in the case of batch-type base catalyzed processes. Alternatively such catalysts may be tableted or extruded in any conventional manner desired to form particles which are wear and impact resistant and can function more effectively in commercial continuous fixed bed type catalyzed processes. However, it is to be understood that in their tableted or extruded form such catalyst particles will be subject to diffusion effects which may negatively impact on the catalyst performance (e.g. product selectivity and catalyst life).

More preferably the calcined magnesium/aluminum mixed oxide catalysts of this invention are obtained by (a) hydrothermally treating the above-described aqueous mixtures or pastes of said water-insoluble magnesium and aluminum salts at a temperature of from about 80°C to about 250°C for a sufficient period of time to obtain a hydrotalcite type mixed magnesium/aluminum hydroxide carbonate catalyst precursor, (b) formulating said catalyst precursor into shaped particles, and (c) drying and calcining the particulate shaped catalyst precursor.

This particular preferred procedure for obtaining calcined magnesium/aluminum mixed oxide catalysts affords one with a far simpler method for producing a hydrotalcite type mixed metal hydroxide carbonate precursor, than presently advocated in the prior art, while at the same time providing a far simpler method for directly obtaining the ultimately more desired particulate shaped calcined magnesium/aluminum mixed oxide catalysts. Moreover it is submitted that the simplicity of this particular preferred procedure of the subject invention facilitates replication of any particular desired calcined magnesium/aluminum mixed oxide catalyst.

The hydrothermal treatment of the above defined aqueous mixtures or pastes of said water-insoluble magnesium and aluminum salts of this invention is carried out for a sufficient period of time in order to convert the aqueous pastes into a hydrotalcite type mixed magnesium/aluminum hydroxide carbonate catalyst precursor, which may be evidenced by the initiation of metal oxygen metal bond formation, as seen by the diminishing X-ray diffraction pattern of the discrete magnesium carbonate salt employed and the beginning of at least the partial appearance of a hydrotalcite type X-ray diffraction pattern within the hydrothermal treated paste. While the hydrothermal treatment may be carried out in any suitable manner, it is recommended and preferred to carry out said treatment in an open environment so as to freely and simultaneously evolve both water and carbon dioxide from the aqueous starting material (e.g. paste) in order to obtain the more preferred calcined catalysts, i.e., those which may provide the best degree of desired aminoether product selectivity and longest catalyst life. Thus it is recommended to avoid hydrothermal treatment conditions that do not simultaneously evolve both water and carbon dioxide (e.g. use of a water condenser during said treatment) as well as hydrothermal treatments in which the temperatures employed are not sufficient enough to simultaneously evolve both water and carbon dioxide from the aqueous paste starting material (e.g. merely air drying). Accordingly, the preferred hydrothermal treatment temperatures employable herein may range from about 100°C to about 175°C and more preferably from about 110°C to about 150°C.

Moreover, while the hydrotalcite type catalyst precursors of this invention could be directly calcined in the same manner as described above for directly calcining the aqueous paste starting materials of this invention, such a procedure would again result in a calcined catalyst in powder form, and as noted above it is more preferred to obtain and/or employ the calcined catalyst as a shaped particulate in continuous catalyzed process operations that require a particulate fixed catalyst bed in order to be commercially attractive.

It is to be understood that while the hydrothermal treatment conditions necessary in order to achieve the most desired optimum results may depend, to a large extent on the experience of the operator of said procedure, such preferred hydrothermal treatment conditions may be readily ascertainable by following the teachings of this invention and routine experimentation. For example one preferred hydrothermal heat treatment involves the passing of the above described aqueous paste starting materials of this invention through a steam heated thermal screw at about 150°C until hydrotalcite type, readily extrudable catalyst precursors are obtained.

The hydrotalcite type catalyst precursors of this invention can be readily formulated into any desired particulate shaped materials, such as tablets (including pills, pellets, etc.), extrudates, and the like. For instance said catalyst precursors can be formulated into conventional extrusions, e.g. 1/8" or 1/16" extrusions, via any conventional extruder. Alternatively, said precursors can be formulated into conventional tablets, e.g. 1/8" by 1/8" tablets, via any conventional molding technique, such as by mixing the catalyst precursor with a small amount, e.g. 0.5 to 2 weight percent, of a suitable mold releasing lubricant, e.g graphite, and the like, and punching out the desired tablets. The mold releasing lubricant is of course removed (burned off) from the catalyst during calcination. In general it is preferred to employ the calcined catalysts herein in their extruded form, when used for the preparation of aminoethers.

Finally, the formulated particulate shaped hydrotalcite type catalyst precursors of this invention are dried and calcined in any conventional known manner, such as already described herein and as disclosed e.g. in U. S. Patent 5,214,142.

The intermediate drying step of the particulate shaped material just prior to calcination is merely a conventional mild heating procedure that is commonly carried out in the art to gradually preheat the moist particulate shaped materials prior to subjecting them to the higher temperatures of calcination, in order to help prevent the non-calcined particulate materials from experiencing too severe a thermal shock, i.e., rupturing or disintegration of the particulate shaped materials due to too rapid a water and/or gas loss caused by the high calcination temperatures. Accordingly, any suitable predrying procedure may be employed herein such as by placing the particulate shaped non-calcined materials in a drying oven and drying them for several hours, e.g. from 2 to 24 hours, at temperatures of about 80°C to about 250°C. More preferably such temperatures may range from about 100°C to about 200°C., while a drying temperature of about 150°C for 2 to 12 hours should be suitable for most purposes.

The calcination of said dried particulate shaped materials may be carried out in any conventional manner desired, such as already discussed herein above. As previously noted calcination temperatures of from about 250°C to about 800°C may be employed, while preferred calcination temperatures preferably range from about 300°C to about 500°C. Again the calcination (or final heat) treatment may be carried out in any conventional furnace or oven such as a muffle furnace, or in air, or an inert gas or even under vacuum and in any conventional known manner desired.

While calcination of hydrotalcite type mixed metal hydroxides catalyst precursors is known to produce mixed metal oxide catalysts of an amorphous nature, it is to be understood that the calcined magnesium/aluminum mixed oxide catalysts of this invention are believed to also still contain some hydroxide and/or carbonate anions in view of their presence in the water-insoluble salts of the aqueous paste starting materials and/or hydrotalcite type mixed metal complex catalyst precursors of this invention.

The calcined magnesium/aluminum mixed oxide catalysts of this invention are especially suitable for use in the production of isophorone and bis(2-dimethylaminoethyl) ether.

The use of such catalysts of this invention for the production of isophorone is considered to be novel and is the subject of assignee's concurrently filed U. S. patent application, Serial No. 8-112,092 entitled "Preparation of Isophorone," (Assignee's Docket No. 17099), the entire application of which is encompassed herein by reference thereto.

Methods for the production of aminoethers and in particular bis(2-dimethylaminoethyl ether are well known and a preferred basic process for preparing such compounds may be found readily described in USP 5,214,142, the entire disclosure of which is incorporated herein by reference thereto.

For instance, carboxylated aminoether compounds can be readily decarboxylated into their desired aminoethers. Thus an additional aspect of this invention relates to a process for producing aminoethers which comprises contacting a carboxylated aminoether compound with a calcined magnesium/aluminum mixed oxide catalyst of this invention, under decarboxylation conditions effective to produce the corresponding aminoether.

Such decarboxylations may be effected in a liquid or vapor or supercritical liquid state or mixtures thereof, although a vapor phase reaction is preferably employed. Further, while such decarboxylation reaction conditions may range from subatmospheric or atmospheric to superatmospheric conditions, it is generally desirable to run the reaction from about 1mm Hg to about 5,000 mm Hg, preferably from about 100 mm Hg to about 2,500 mm Hg.

The temperature of the decarboxylation reaction may range from about 150°C to about 500°C. Preferably, the reaction temperature ranges from about 175°C to about 375°C, and most preferably from about 225°C to about 350°C.

Suitable carboxylated aminoether compounds useful as starting materials in such decarboxylation reactions, as well as methods for their preparation, are well known in the art and include bis[2-(N,N-dialkylamino)alkyl]carbonates, wherein each alkyl radical may independently contain from 1 to 4 carbon atoms, and the like.

The decarboxylation reaction can be conducted in the presence of an inert diluent which can be either a liquid or gas. Examples of useful liquid diluents include benzene, toluene, xylene, ethylbenzene, anisole, heptane, octane, nonane, decane, dibutyl ether, and the like. Hydrocarbons are preferred. Illustrative of gaseous diluents include, for example, nitrogen, methane, hydrogen, carbon monoxide or carbon dioxide. The gaseous diluent should of course be chosen so that it does not prevent the preparation of the desired aminoether products.

While the use of such diluents may be beneficial, the decarboxylation process can be operated using a pure starting material liquid or gaseous feed. The degree of dilution of the starting material with various diluents may vary considerably depending only upon any process constraints restricting the use of the diluent. Thus the amount of liquid diluent can vary widely, for instance, from no diluent to about 90 weight percent or greater of the total weight of the starting material/carrier feed.

The calcined magnesium/aluminum mixed oxide catalysts may be employed in such decarboxylations with or without a support, binder or other additive to help stabilize the catalyst. The amount of catalyst employed need only be a catalytic amount and is dependent in general only on the particular catalyst and decarboxylation procedure employed. In general if a batchwise process is employed the catalyst employed can range from about 0.01 weight percent or less to about 10 weight percent or greater of the total weight of the starting materials. If a continuous or vapor phase process is employed, any conventional fixed or fluid type bed of the catalyst may be employed.

The decarboxylation process of this invention is useful for preparing a large variety of conventional aminoethers, such as bis[2-(N,N-dialkylamino)alkyl]ethers, wherein each alkyl radical may independently contain from 1 to 4 carbon atoms, and the like, and is especially useful for selectively preparing bis[2-(N,N-dimethylamino)ethyl]ether, which is a well known urethane catalyst, from bis[2-(N,N-dimethylamino)ethyl] carbonate. The aminoether products so produced can be separated and recovered from any product mixture by conventional distillation techniques.

The decarboxylation reaction may be carried out using, for example, a fixed bed reactor, a fluid bed reactor, or a slurry reactor. In general, for fluid bed reactors, a small, spherical catalyst particle is preferred for easy fluidization. With fixed bed reactors, larger catalyst particles are preferred so the back pressure within the reactor is kept reasonably low.

Moreover as noted the decarboxylation process of this invention can be conducted in a batch or continuous fashion using any suitable conventional reaction equipment and technique.

Further, the decarboxylation process is conducted for a period of time sufficient to produce the desired aminoethers. The exact reaction time employed is dependent, in part, upon factors such as temperature, nature and proportion of starting materials, and the like. The reaction time will normally be within the range of from about one-half to about 100 hours or more, and preferably from less than about one to about ten hours.

The following examples are illustrative of the present invention and are not to be regarded as limitative. It is to be understood that all parts, percentages and proportions referred to herein and in the appended claims are by weight unless otherwise indicated.

## Catalyst Preparations

### Example 1

A total of 44.1 grams of magnesium nitrate hexahydrate and 66.0 grams of aluminum nitrate nonahydrate were dissolved in 200 ml. of distilled water to give solution A.

A total of 4.8 grams of ammonium carbonate was dissolved in 200 ml. of concentrated ammonium hydroxide (28-29 wt. percent) to give solution B.

100 ml. of distilled water was heated in a flask at 40°C. and solutions A and B were combined simultaneously with good agitation using a mechanical stirrer. The rates of addition of solution A and B were adjusted to maintain a pH of 9-10. The total addition took 10 minutes and a final pH of 9.5 was obtained. The contents were stirred at 40°C. for 40 minutes. The precipitate was filtered and washed with about sequential amounts of about 300 ml. of water at 60°C. until the pH of the wash was neutral. The filter cake was dried at 80°C. overnight and then calcined in air at 400°C. for 3 hours to afford a calcined magnesium/aluminum mixed oxide catalyst having a Mg/Al oxide molar ratio of about 2:1. This comparative catalyst is referred to hereinafter as Catalyst A.

### Example 2

A total of about 6.0 grams of magnesium carbonate hydroxide pentahydrate from Aldrich Chemical Co., Inc. and about 6.0 grams of aluminum hydroxide carbonate hydrate (aluminum hydroxide gel, dried from Chattem Chemicals) were combined in a porcelain dish and mixed well with a spatula. A total of 30 grams of distilled water was then added at one time to form a paste. (Initially the mixture is thick, but after mixing for about two minutes a smooth paste is formed.) The aqueous paste mixture was immediately calcined at 400°C. for 6 hours to give about 6.2 grams of calcined magnesium/aluminum mixed oxide catalyst having a Mg/Al oxide molar ratio of about 2:1. The catalyst is referred to hereinafter as Catalyst B.

### Example 3

A total of about 8.0 grams of magnesium carbonate hydroxide pentahydrate from Aldrich Chemical Co., Inc., and about 4.0 grams of aluminum hydroxide carbonate hydrate (aluminum hydroxide hydrate, powder from Aldrich Chemical Co., Inc.) were combined in a porcelain dish and mixed well with a spatula. A total of 36 grams of distilled water was then added at one time to form a paste. The aqueous paste mixture was immediately calcined at 350°C. for 3 hours to give about 7.3 grams of calcined magnesium/aluminum mixed oxide catalyst having a Mg/Al oxide molar ratio of about 4:1. The catalyst is referred to hereinafter as Catalyst C.

Example 4

A total of about 5.10 grams of anhydrous magnesium carbonate from Eastman Fine Chemicals and about 6.0 grams of aluminum hydroxide carbonate hydrate (aluminum hydroxide hydrate, powder from Aldrich Chemical Co., Inc.) were combined in a porcelain dish and mixed well with a spatula. A total of 24 grams of distilled water was then added at one time to form an aqueous paste, which was then heated at 150°C. for three hours to form a catalyst precursor. The catalyst precursor was then calcined at 400°C. overnight to give about 4.9 grams of calcined magnesium/aluminum mixed oxide catalyst having a Mg/Al oxide molar ratio of about 2:1. The catalyst is referred to hereinafter as Catalyst D.

Example 5

A total of about 6.10 grams of magnesium carbonate monohydrate from J. T. Baker Inc. and about 6.0 grams of aluminum hydroxide carbonate hydrate (aluminum hydroxide hydrate, powder from Aldrich Chemical Co., Inc.) were combined in a porcelain dish and mixed well with a spatula. A total of 24 grams of distilled water was then added at one time to form an aqueous paste, which was then heated at 150°C. overnight to form a catalyst precursor. The catalyst precursor was then calcined at 400°C. for four hours to give about 5.4 grams of calcined magnesium/aluminum mixed oxide catalyst having a Mg/Al oxide molar ratio of about 2:1. The catalyst is referred to hereinafter as Catalyst E.

Example 6

A total of about 5.90 grams of magnesium carbonate hydroxide trihydrate from Pfaltz and Bauer Inc. and about 6.0 grams of aluminum hydroxide carbonate hydrate (aluminum hydroxide hydrate, powder from Aldrich Chemical Co., Inc.) were combined in a porcelain dish and mixed well with a spatula. A total of 22 grams of distilled water was then added at one time to form an aqueous paste, which was then heated at 150°C. overnight to form a catalyst precursor. The catalyst precursor was then calcined at 400°C. for four hours to give about 5.2 grams of calcined magnesium/aluminum mixed oxide catalyst having a Mg/Al oxide molar ratio of about 2:1. The catalyst is referred to hereinafter as Catalyst F.

Examples 7 to 12

Preparation of Bis[2-(N,N-dimethylamino)ethyl]ether

In the examples set forth in Tables I to III below, an Applied Test Systems, Inc. Model 3620 Split Test Oven equipped with a preheater (stainless steel 1/8 inch o.d. x 2 feet) and 1/2 inch (o.d.) stainless steel reactor tube (8 inch length) was packed with catalyst and heated to the desired reaction temperature using a Honeywell Dial-A-Trol temperature controller. The temperatures at the top of the reactor and the bottom were monitored using a digital temperature readout. The liquid feed of neat bis[2-(N,N-dimethylamino)ethyl]carbonate was decarboxylated by contacting it with a calcined magnesium/aluminum mixed oxide catalyst by passing it through a liquid preheater and adding it (downflow) to the reactor containing said catalyst via a Fluid Metering Inc. RP-G20 drive pump equipped with an 1/8 inch pump head. The system was maintained under nitrogen, which was introduced prior to the liquid preheater and was monitored with a rotameter. The product mixture was collected in a 100 milliliter round bottom flask, vented first to a dry ice/acetone trap and then a Firestone valve. Analysis was performed by capillary gas chromatography (FID) using a DB-1701 column. The process conditions including the particular catalyst used and product analyses are set forth in Tables I to III.

7

## Table I

| Example No. | 7 | 7 | 7 | 8 | 8 | 8 | 8 |
|---|---|---|---|---|---|---|---|
| **Process Parameters** | | | | | | | |
| Catalyst Used | A | A | A | B | B | B | B |
| Catalyst Weight, gm. | 6.0 | 6.0 | 6.0 | 3.0 | 3.0 | 3.0 | 3.0 |
| Liquid Feed Rate, ml/min. | 0.30 | 0.32 | 0.32 | 0.32 | 0.31 | 0.30 | 0.32 |
| $N_2$ Gas Feed Rate, ml/min. | 37 | 37 | 37 | 37 | 37 | 37 | 37 |
| Temperature, °C | 300 | 300 | 300 | 300 | 300 | 300 | 300 |
| Accumulated Time on Stream, hrs. | 2 | 12 | 26 | 2 | 12 | 26 | 40 |
| **Product Composition, area %** | | | | | | | |
| 2-(N,N-dimethylamino)ethanol | 4.39 | 2.92 | 1.87 | 3.41 | 3.64 | 3.24 | 3.09 |
| N,N,N',N'-tetramethylethylenediamine | 11.43 | 9.76 | 5.94 | 11.80 | 13.98 | 13.99 | 13.42 |
| Bis[2-(N,N-dimethylamino)ethyl]ether | 76.75 | 60.42 | 33.67 | 74.49 | 74.79 | 73.41 | 68.77 |
| Bis[2-(N,N-dimethylamino)ethyl]carbonate | 0.61 | 23.12 | 56.59 | 0.00 | 0.89 | 3.89 | 9.88 |
| Others | 6.82 | 3.78 | 1.93 | 10.30 | 6.70 | 5.47 | 4.84 |

EP 0 640 388 A1

Table II

| Example No. | 9 | 9 |
|---|---|---|
| **Process Parameters** | | |
| Catalyst Used | C | C |
| Catalyst Weight, gm. | 3.0 | 3.0 |
| Liquid Feed Rate, ml/min. | 0.28 | 0.29 |
| N$_2$ Gas Feed Rate, ml/min. | 37 | 37 |
| Temperature, °C | 300 | 300 |
| Accumulated Time on Stream, hr. | 2 | 14 |
| | | |
| **Production Composition, area %** | | |
| 2-(N,N-dimethylamino)ethanol | 3.74 | 3.31 |
| N,N,N',N'-tetramethylethylenediamine | 10.52 | 12.68 |
| Bis[2-(N,N-dimethylamino)ethyl]ether | 76.28 | 69.52 |
| Bis[2-(N,N-dimethylamino)ethyl]carbonate | 0.00 | 9.28 |
| Others | 9.46 | 5.21 |

## Table III

| Example No. | 10 | 10 | 11 | 11 | 12 | 12 |
|---|---|---|---|---|---|---|
| **Process Parameters** | | | | | | |
| Catalyst Used | D | D | E | E | F | F |
| Catalyst Weight, gm. | 3.0 | 3.0 | 3.0 | 3.0 | 3.0 | 3.0 |
| Liquid Feed Rate, ml/min. | 0.34 | 0.33 | 0.33 | 0.34 | 0.33 | 0.32 |
| $N_2$ Gas Feed Rate, ml/min. | 37 | 37 | 37 | 37 | 37 | 37 |
| Temperature, °C | 325 | 325 | 325 | 325 | 325 | 325 |
| Accumulated Time, on Stream hrs. | 3 | 20 | 2 | 15 | 6 | 18 |
| | | | | | | |
| **Product Composition, area %** | | | | | | |
| 2-(N,N-dimethylamino)ethanol | 3.67 | 4.29 | 2.97 | 4.04 | 3.54 | 3.94 |
| N,N,N',N'-tetramethylethylenediamine | 14.05 | 17.94 | 13.39 | 17.70 | 14.34 | 17.31 |
| Bis[2-(N,N-dimethylamino)ethyl]ether | 70.04 | 68.27 | 70.41 | 69.43 | 71.67 | 69.85 |
| Bis[2-(N,N-dimethylamino)ethyl]carbonate | 0.00 | 2.09 | 0.00 | 1.30 | 0.00 | 1.24 |
| Others | 12.24 | 7.41 | 13.23 | 7.53 | 10.45 | 7.66 |

The results in above Tables I to III demonstrate the superior performance of the catalysts of this invention which employed various carbonate containing magnesium salts in their preparation (i.e. Catalysts B to F) over that of precipitated comparative Catalyst A for selectively producing bis[2-N,N-(dimethylamino)ethyl]ether over a prolonged period of time.

### Example 13

A total of about 10.0 grams of a mixture consisting of equal parts by weight of magnesium carbonate hydroxide pentahydrate and aluminum hydroxide carbonate hydrate powders was combined with 20 grams of distilled water to form a paste which was dried at 150°C overnight, and calcined in air at 400°C for 6 hours to give about 5.1 grams of calcined magnesium/aluminum mixed oxide catalyst having a Mg/Al oxide molar ratio of about 2:1. The catalyst is referred to hereinafter as Catalyst J.

### Example 14

A total of about 15.0 grams of a mixture consisting of equal parts by weight of magnesium carbonate hydroxide pentahydrate and of aluminum hydroxide carbonate hydrate powders was combined with about 30 grams of distilled water to form a paste, and allowed to dry at ambient temperature overnight. The material was then calcined in air at 400°C for 6 hours to give about 7.7 grams of calcined magnesium/aluminum mixed oxide catalyst having a Mg/Al oxide molar ratio of about 2:1. The catalyst is referred to hereinafter as Catalyst K.

### Example 15

A total of about 10.0 grams of a mixture consisting of equal parts by weight of magnesium carbonate hydroxide pentahydrate and aluminum hydroxide carbonate hydrate powders was combined with about 40 grams of distilled water to form a paste, and added to a 100 ml. three-neck round bottom flask equipped with a water condenser. This mixture was heated at 45-95°C for 5.5 hours, cooled and the flask placed in an oven at 150°C overnight. This material was then calcined in air at 400°C for 4 hours to give about 5.3 grams of calcined magnesium/aluminum mixed oxide catalyst having a Mg/A1 oxide molar ratio of about 2:1. The catalyst is referred to hereinafter as Catalyst L.

### Examples 16 to 18

Preparation of Bis[2-(N,N-dimethylamino)ethyl]ether

In the examples set forth in Table IV below, bis[2-(N,N-dimethylamino)ethyl]carbonate was decarboxylated by contacting it with a calcined magnesium/aluminum mixed oxide catalyst to produce bis[2-(N,N-dimethylamino)ethyl]ether in the same manner as described above for Examples 7 to 12. The process conditions, catalysts employed and product analyses are set forth in said Table IV.

Table IV

| Example No. | 16 | 16 | 16 | 17 | 17 | 18 | 18 |
|---|---|---|---|---|---|---|---|
| **Process Parameters** | | | | | | | |
| Catalyst Used | J | J | J | K | K | L | L |
| Catalyst Weight, gm. | 3.0 | 3.0 | 3.0 | 3.0 | 3.0 | 3.0 | 3.0 |
| Liquid Feed Rate, ml/min. | 0.31 | 0.31 | 0.32 | 0.33 | 0.34 | 0.34 | 0.35 |
| N$_2$ Gas Feed Rate, ml/min. | 37 | 37 | 37 | 37 | 37 | 37 | 37 |
| Temperature, °C | 325 | 325 | 325 | 325 | 325 | 325 | 325 |
| Accumulated Time on Stream, hrs. | 6.0 | 26.0 | 43.0 | 11 | 23 | 7 | 19 |
| | | | | | | | |
| **Product Composition, GC Anal., area %** | | | | | | | |
| 2-(N,N-dimethylamino)ethanol | 2.92 | 3.53 | 4.09 | 3.89 | 4.23 | 3.32 | 3.39 |
| N,N,N',N'-tetramethylethylenediamine | 14.92 | 17.95 | 19.87 | 16.44 | 19.41 | 14.45 | 17.31 |
| Bis[2-(N,N-dimethylamino)ethyl]ether | 73.65 | 71.38 | 68.62 | 71.73 | 68.58 | 74.82 | 71.60 |
| Bis[2-(N,N-dimethylamino)ethyl]carbonate | 0.00 | 0.00 | 0.84 | 0.00 | 0.90 | 0.00 | 1.06 |
| Others | 8.51 | 7.14 | 6.58 | 7.94 | 6.88 | 7.41 | 6.64 |

The results in above Table IV demonstrate the desirability of employing a hydrothermal treatment that si-

12

multaneously evolves both water and carbon dioxide when preparing the catalysts of this invention (e.g. Catalyst J) as opposed to employing a hydrothermal treatment that is not sufficient to simultaneously evolve both water and carbon dioxide (e.g. in the preparation of Catalyst K only water was evolved, while in the preparation of Catalyst L only carbon dioxide was evolved) as seen by their comparative productions of bis[2-N,N-(dimethylamino)ethyl] ether over prolonged periods of time.

Example 19

A total of 1000 grams of magnesium carbonate hydroxide pentahydrate from Aldrich Chemical Co., Inc., and 1000 grams of aluminum hydroxide carbonate hydrate (aluminum hydroxide hydrate, powder from Aldrich Chemical Co., Inc.) along with 5000 grams of deionized water were mixed together to form a slurry in an Eirich mixer. The slurry was then hydrothermally treated in a thermal screw at 150°C and the resulting paste was formed into 1/8 inch extrudates in an extruder. The extrudates were dried at 150°C in an oven, and then placed in a calcination oven and calcined at 400°C for 6 hours to give a calcined magnesium/aluminum mixed oxide catalyst have a Mg/Al oxide molar ratio of about 1.9:1. The catalyst is referred to hereinafter as Catalyst M.

Examples 20

Preparation of Bis[2-(N,N-dimethylamino)ethyl]ether

In the example set forth in Table V below, bis[2-(N,N-dimethylamino)ethyl]carbonate was decarboxylated by contacting it with a calcined magnesium/aluminum mixed oxide catalyst to produce bis[2-(N,N-dimethylamino)ethyl]ether in the same manner as described above for Examples 16 to 18. The process conditions, catalyst employed and product analyses are set forth in said Table V.

## Table V

| Example No. | 20 | 20 | 20 | 20 | 20 | 20 | 20 |
|---|---|---|---|---|---|---|---|
| **Process Parameters** | | | | | | | |
| Catalyst Used | M | M | M | M | M | M | M |
| Catalyst Weight, gm. | 3.0 | 3.0 | 3.0 | 3.0 | 3.0 | 3.0 | 3.0 |
| Liquid Feed Rate, ml/min. | 0.33 | 0.30 | 0.31 | 0.30 | 0.33 | 0.32 | 0.30 |
| $N_2$ Gas Feed Rate, ml/min. | 37 | 37 | 37 | 37 | 37 | 37 | 37 |
| Temperature, °C | 325 | 325 | 325 | 325 | 325 | 325 | 325 |
| Accumulated Time on Stream, hrs. | 1.0 | 3.5 | 7.0 | 11.0 | 14.0 | 18.0 | 21.0 |
| **Product Composition, GC Anal., area %** | | | | | | | |
| 2-(N,N-dimethylamino)ethanol | 2.94 | 4.08 | 3.98 | 3.88 | 3.72 | 3.52 | 3.52 |
| N,N,N'-tetramethylethylenediamine | 10.76 | 13.02 | 14.46 | 15.69 | 15.95 | 15.85 | 15.93 |
| Bis[2-(N,N-dimethylamino)ethyl]ether | 74.34 | 75.57 | 75.38 | 73.90 | 72.99 | 71.40 | 70.16 |
| Bis[2-(N,N-dimethylamino)ethyl]carbonate | 0.00 | 0.04 | 0.40 | 1.15 | 2.42 | 5.07 | 6.87 |
| Others | 11.96 | 7.29 | 5.78 | 5.38 | 4.92 | 4.16 | 3.52 |

The results in above Table V demonstrate the excellent performance of an extrudated catalyst of this invention (Catalyst M) for selectively producing bis[2-N,N-(dimethylamino)ethyl]ether over a prolonged period of time.

Example 21

A total of about 3.20 grams of magnesium oxide light from Aldrich Chemical Co., Inc. and about 8.02 grams of aluminum hydroxide carbonate hydrate (aluminum hydroxide hydrate, powder from Aldrich Chemical Co.,

Inc.) were combined in a porcelain dish and mixed well with a spatula. A total of 22 grams of distilled water was then added at one time to form an aqueous paste, which was then heated at 150°C for 4 hours to form a catalyst precursor. The catalyst precursor was then calcined at 400°C overnight to give about 8.1 grams of calcined magnesiuim/aluminum mixed oxide catalyst having a Mg/Al oxide molar ratio of about 2:1. This comparative catalyst is referred to hereinafter as Catalyst N.

Example 22

A total of about 6.0 grams (0.012 mol) of magnesium carbonate hydroxide pentahydrate from Aldrich Chemical Co., Inc. and about 4.4 grams of alumina trihydrate, $Al_2O_3 \cdot 3H_2O$ (H-30 from LaRoche Chemicals, Inc.) were combined with about 20 grams of distilled water and mixed to form an aqueous paste. The paste was then calcined overnight at 350°C to give about 6.15 grams of a calcined magnesium/aluminum mixed oxide catalyst having a Mg/Al oxide molar ratio of about 2:1. This comparative catalyst is referred to hereinafter as Catalyst O.

Examples 23 to 24

Preparation of Bis[2-(N,N-dimethylamino)ethyl]ether

In the examples set forth in Table VI below, bis[2-(N,N-dimethylamino)ethyl]carbonate was decarboxylated by contacting it with a calcined magnesium/aluminum mixed oxide catalyst to produce bis[2-(N,N-dimethylamino)ethyl]ether in the same manner as described above for Example 20. The process conditions, catalysts employed and product analyses are set forth in said Table VI.

15

**Table VI**

| Example No. | 23 | 23 | 24 | 24 |
|---|---|---|---|---|
| **Process Parameters** | | | | |
| Catalyst Used | N | N | O | O |
| Catalyst Weight, gm. | 3.0 | 3.0 | 3.0 | 3.0 |
| Liquid Feed Rate, ml/min. | 0.35 | 0.35 | 0.25 | 0.28 |
| $N_2$ Gas Feed Rate, ml/min. | 37 | 37 | 37 | 37 |
| Temperature, °C | 325 | 325 | 300 | 300 |
| Accumulated Time on Stream, hrs. | 5.0 | 9.0 | 0.5 | 1.0 |
| **Product Composition, GC Anal., area %** | | | | |
| 2-(N,N-dimethylamino)ethanol | 5.48 | 6.54 | 24.45 | 13.54 |
| N,N,N',N'-tetramethylethylenediamine | 15.13 | 17.33 | 18.56 | 12.18 |
| Bis[2-(N,N-dimethylamino)ethyl]ether | 66.90 | 63.56 | 11.93 | 6.68 |
| Bis[2-(N,N-dimethylamino)ethyl]carbonate | 0.19 | 2.28 | 17.76 | 54.71 |
| Others | 12.30 | 10.29 | 27.30 | 12.89 |

The results of the above Table VI demonstrate the poor catalyst performance in terms of selectivity for producing bis[2-(N,N-dimethylamino)ethyl]ether and catalyst life when either the magnesium salt or the aluminum salt from which the catalyst was derived did not contain any carbonate, i.e. comparative catalysts N and O.

Various modifications and variations of this invention will be obvious to a worker skilled in the art and it is to be understood that such modifications and variations are to be included within the purview of this application and the spirit and scope of the appended claims.

## Claims

1. A calcined magnesium/aluminum mixed oxide catalyst derived from an aqueous mixture consisting of from about 0.1 to about 5 parts by weight of a water-insoluble magnesium carbonate selected from magnesium carbonate hydroxide pentahydrate, anhydrous magnesium carbonate, magnesium carbonate monohydrate, or magnesium carbonate hydroxide trihydrate, per one part by weight of a water-insoluble aluminum hydroxide carbonate hydrate, and from about 1 to about 10 parts by weight of water per one part of the total weight of the water-insoluble aluminum and magnesium compounds employed.

2. A catalyst as claimed in claim 1, wherein the aqueous mixture consists of from about 1 to about 3 parts by weight of the water-insoluble magnesium carbonate per one part by weight of the water-insoluble aluminum hydroxide carbonate hydrate, and from about 1 to about 3 parts by weight of water per one part of the total weight of the water-insoluble aluminum and magnesium compounds employed.

3. A catalyst as claimed in claim 1 or claim 2, wherein the magnesium carbonate is magnesium carbonate hydroxide pentahydrate.

4. A catalyst as claimed in claim 1 wherein the catalyst is obtained by (a) hydrothermally treating the aqueous mixture of the water-insoluble magnesium and aluminum salts at a temperature of from about 80°C to about 250°C for a sufficient period of time to obtain a hydrotalcite type mixed magnesium/aluminum hydroxide carbonate catalyst precursor, (b) formulating said catalyst precursor into shaped particles, and (c) drying and calcining the particulate shaped catalyst precursor.

5. A catalyst as claimed in claim 2 or claim 3 wherein the catalyst is obtained by (a) hydrothermally treating the aqueous mixture of the water-insoluble magnesium and aluminum salts at a temperature of from about 80°C to about 250°C for a sufficient period of time to obtain a hydrotalcite type mixed magnesium/aluminum hydroxide carbonate catalyst precursor, (b) formulating said catalyst precursor into shaped particles, and (c) drying and calcining the particulate shaped catalyst precursor.

6. A process for producing aminoethers which comprises contacting a carboxylated aminoether compound with a calcined magnesium/aluminum mixed oxide catalyst derived from an aqueous mixture consisting of from about 0.1 to about 5 parts by weight of a water-insoluble magnesium carbonate selected from magnesium carbonate hydroxide pentahydrate, anhydrous magnesium carbonate, magnesium carbonate monohydrate, or magnesium carbonate hydroxide trihydrate, per one part by weight of a water-insoluble aluminum hydroxide carbonate hydrate, and from about 1 to about 10 parts by weight of water per one part of the total weight of the water-insoluble aluminum and magnesium compounds employed under decarboxylation conditions effective to produce the corresponding aminoether.

7. A process as claimed in claim 6, wherein the aqueous mixture consists of from about 1 to about 3 parts by weight of the water-insoluble magnesium carbonate per one part by weight of the water-insoluble aluminum hydroxide carbonate hydrate, and from about 1 to about 3 parts by weight of water per one part of the total weight of the water-insoluble aluminum and magnesium compounds employed.

8. A process as claimed in claim 6 or claim 7 wherein the magnesium carbonate hydroxide pentahydrate.

9. A process as claimed in claim 6 or claim 7 wherein bis[2-(N,N-dimethylamino)ethyl]ether is prepared from bis[2-(N,N-dimethylamino)ethyl] carbonate.

10. A process as claimed in any one of claims 6 to 9 wherein the catalyst is obtained by (a) hydrothermally treating the aqueous mixture of the water-insoluble magnesium and aluminum salts at a temperature of from about 80 to about 250°C for a sufficient period of time to obtain a hydrotalcite type mixed magnesium/aluminum hydroxide carbonate catalyst precursor, (b) formulating the catalyst precursor into shaped particles, and (c) drying and calcining the particulate shaped catalyst precursor.

| European Patent Office | EUROPEAN SEARCH REPORT | Application Number |
|---|---|---|
| | | EP 94 30 6228 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | EP-A-0 476 785 (UNION CARBIDE)<br>* page 4, line 26 - line 40 *<br>* page 7, line 41 - line 56 *<br>* page 8, line 54 - line 57 *<br>* page 18; example 13 *<br>* claims *<br>--- | 1-10 | B01J21/10<br>C07C213/06<br>B01J37/08 |
| X | EP-A-0 478 075 (UNION CARBIDE)<br>* page 4, line 9 - line 18 *<br>* page 7, line 15 - line 18 *<br>* page 16; example 13 *<br>* claims *<br>--- | 1-10 | |
| A | WO-A-90 12645 (ARISTECH)<br>* page 3, paragraph 2 -paragraph 3 *<br>* page 5, paragraph 2 *<br>* page 7 - page 8; examples 1,2 *<br>* claims *<br>--- | 1-10 | |
| A | EP-A-0 479 363 (UNION CARBIDE)<br>----- | | TECHNICAL FIELDS SEARCHED (Int.Cl.6)<br><br>B01J<br>C07C |

The present search report has been drawn up for all claims

| Place of search | Date of completion of the search | Examiner |
|---|---|---|
| THE HAGUE | 7 December 1994 | LO CONTE, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)